# FASCICULE DE BREVET EUROPEEN

(11) **EP 0 388 422 B1**
(45) Date de publication et mention de la délivrance du brevet: **23.06.1993**
(21) Numéro de dépôt: 88910061.6
(22) Date de dépôt: 23.11.1988
(51) Int. Cl.: A61B 5/08

(54) **PROCEDE ET DISPOSITIF DE SURVEILLANCE DE LA RESPIRATION D'UN INDIVIDU**
VERFAHREN UND VORRICHTUNG ZUR ATMUNGSÜBERWACHUNG BEI EINER PERSON
PROCESS AND DEVICE FOR MONITORING HUMAN RESPIRATION

(30) Priorité: 23.11.1987 FR 8716174
(43) Date de publication de la demande: 26.09.1990
(73) Titulaire: BERTIN & CIE, F-78373 Plaisir Cédex (FR)
(72) Inventeur: GUERN, Yves, François, Charles, Quartier du Couvent F-83470 Pourrières (FR); WEBER, Jean-Luc, Michel, F-13300 Salon-de-Provence (FR); TOMMASI, Marc, Raymond, F-13001 Marseille (FR)
(74) Mandataire: de Boisse, Louis Arnaud
(86) Numéro de dépôt international: FR8800570
(87) Numéro de publication internationale: WO8904633

(56) Documents cités:
- FR-A- 2 141 047
- US-A- 4 350 166
- Medical & Biological Engineering & Computing, vol. 24, No: 2, March 1986, (Stevenage, Herts, GB), H. Teichtahl et al.: "Measurement of in vitro ciliary beat frequency: a television-video modification of the transmitted light technique", pages 193-196
- IEEE Transactions on Bio-Medical Engineering, vol. 24, No: 1, January 1977, T.G. Uter: "A real-time video system for tracking one-dimensional movements of two objects", pages 75-78
- Proceedings of MELECON '85, Madrid, 8-10 October 1985, vol. 1, Elsevier Science Publishers B.V. (North-Holland), V. Macellari et al.: "Comparison among remote sensing systems for human movement measurement", pages 71-75

## Description

L'invention concerne un procédé et un dispositif de surveillance de la respiration d'un individu, notamment en vue de détecter un défaut de respiration, apnée ou dyspnée, durant le sommeil d'un nourrisson ou d'un malade alité.

Il a été constaté de nombreux cas de mort subite de nourrissons ou jeunes enfants âgés de une semaine à deux ans en raison d'un disfonctionnement des automatismes respiratoires. Environ 75% de ces cas d'arrêt respiratoire ne présentent aucun signe prémonitoire.

La détection précoce de ces accidents nécessite donc de surveiller en permanernce la régularité de la respiration des enfants. Pour cela, différents appareils ont été proposés, que l'on peut classer en deux catégories.

Un premier type d'appareil de détection nécessite un contact direct avec le sujet. Ces appareils utilisent un capteur de déplacement qui enregistre les variations de l'ampliation thoracique (jauge d'extensiométrie, capteur à effet Hall, etc...) ou la modification de la répartition des pressions sous le matelas supportant le nouveau-né (capteurs à effet capacitif, résistif ou inductif, ou capteurs de mesure de la pression locale). Les premiers sont peu commodes d'utilisation et sources d'accidents en raison de la liaison permanente qui doit exister entre l'enfant qui sommeille et l'appareil de détection, tandis que les seconds sont coûteux en raison de l'adaptation nécessaire du réseau de capteurs au lit de l'enfant et d'une fiabilité discutable sauf à recourir à des capteurs d'une très grande sensibilité.

Les appareils du second type, ne nécessitant pas de contact direct avec le sujet, présentent l'avantage de ne pas gêner celui-ci dans ses mouvements pendant son sommeil. Ces appareils peuvent être basés sur la détection du souffle expiratoire (thermistance, photodétecteur infrarouge, etc...), mais ils peuvent alors être mis en défaut lors des déplacements importants du sujet (retournement antérieur-postérieur ou latéral). D'autres appareils font appel à des capteurs volumétriques à ultrasons, infrarouges ou autres : ces capteurs sont bien adaptés lorsque la scène est relativement statique, c'est-à-dire que le seul mouvement de l'individu est dû à sa respiration, mais l'information qu'ils délivrent est plus difficile à exploiter lorsque le sujet se déplace dans le champ d'observation.

Parmi les appareils de détection sans contact, on connaît également, par le document FR-A-2 141 047, un appareil de surveillance au moyen duquel le corps de l'individu est soumis à un rayonnement micro-ondes.

Lorsque l'individu respire, la fréquence du rayonnement réfléchi est différente à chaque instant de celle du rayonnement incident en raison du mouvement de la paroi de la cage thoracique et la différence de fréquence entre les ondes émises et les ondes reçues est convertie en un signal représentatif de la vitesse actuelle de la paroi thoracique à chaque instant du cycle respiratoire.

Si le niveau de ce signal tombe au-dessous d'un certain seuil et que cette situation se prolonge au-delà d'un laps de temps prédéterminé, une alarme signalant l'arrêt de la respiration est déclenchée.

L'appareil comporte par conséquent une source de rayonnement micro-ondes qui est active, directive et rayonne dans une bande de fréquences très étroite.

Le recours à la technique des micro-ondes nécessite des appareillages (aériens, circuits électriques d'alimentation et circuits électroniques de traitement) relativement coûteux et gourmands en énergie électrique.

La mise en oeuvre en est délicate et nécessite certaines précautions pour que les résultats obtenus ne soient pas faussés par les mouvements des personnes se déplaçant autour de l'individu sous surveillance et pour que ce dernier ne soit pas soumis à un niveau d'énergie rayonné excessif. En outre, les conséquences d'une exposition prolongée à un rayonnement micro-ondes sont mal connues, en particulier pour les nourrissons.

On connaît encore par le document US-A-4 350 166 un procédé dans lequel on détecte la modulation du rayonnement infra-rouge ambiant par le dioxyde de carbone exhalé par la respiration d'un individu. Les capteurs fournissent donc des signaux modulés et, s'il se produit une interruption dans la périodicité de ces signaux, une alarme est déclenchée.

L'invention vise à fournir un procédé et un appareil de surveillance de la respiration d'un individu qui permettent de s'affranchir des inconvénients de la technique antérieure.

Les facteurs de risques des accidents respiratoires mentionnés précédemment n'étant pas connus, seule une surveillance à très grande échelle des enfants après leur naissance permettrait d'assurer une prévention efficace. Le besoin se fait donc sentir d'un moyen permettant de surveiller, tant en milieu hospitalier qu'à domicile, la respiration de jeunes enfants, de détecter de manière précoce un défaut de respiration, apnée ou dyspnée, et de déclencher une alarme en réponse à une telle détection. Un tel moyen doit être adaptable à toutes les situations de couchage, ne pas nécessiter de réglage, être facile à mettre en oeuvre, et peu coûteux.

A cet effet, l'invention a pour objet un procédé de surveillance de la respiration d'un individu, suivant lequel le corps dudit individu est au moins partiellement soumis à un champ, les perturbations causées au champ par les mouvements de l'individu sont détectées et une alarme est déclenchée lorsque le degré de mouvement perturbant le champ tombe au-dessous d'un seuil prédéterminé pendant un temps supérieur à une valeur prédéterminée, ledit procédé consiste à exposer au moins partiellement le corps dudit individu à un flux lumineux, à former sur un ensemble d'éléments photosensibles l'image d'au moins une zone de l'espace exposée au flux lumineux et comprise dans le champ d'observation d'un moyen optique associé aux éléments photosensibles, ladite zone englobant au moins une partie du corps dudit individu susceptible de présenter des mouvements quasi périodiques liés à la respiration, à mesurer et à enregistrer avec une fréquence de récurrence ou de lecture déterminée les niveaux de gris des points de ladite image, à comparer entre eux les niveaux de gris des points correspondants d'images successives, et à détecter un défaut de respiration, apnée ou dyspnée, dudit individu en l'absence de variations significatives ou en présence de variations anormales de la distribution spatiale des niveaux de gris des points d'images successives comparées.

Le procédé selon l'invention consiste à observer l'individu sous surveillance en présence d'un flux lumineux. Ce flux peut être constitué par la lumière ambiante ou peut être produit par une source lumineuse qui peut présenter un très large spectre du domaine visible ou non et qui n'est pas nécessairement directive puisque seul importe le champ d'observations.

Le procédé de surveillance suivant l'invention est périodique séquentiel (et non continu comme dans le document FR-A-2 147 047) : l'image lumineuse d'une partie du corps de l'individu reçue sur un ensemble d'éléments photosensibles est lue à intervalles de temps réguliers en enregistrant les niveaux de gris des points de cette image.

On obtient ainsi une série d'images enregistrées successives dont on compare deux à deux la distribution spatiale des niveaux de gris : si cette distribution spatiale est la même, c'est que l'individu est immobile et ne respire plus. Cette caractéristique de comparaison de la distribution spatiale des niveaux de gris d'images prises deux à deux est complètement étrangère au document FR-A-2 147 047.

Il est important de noter que le procédé suivant l'invention permet de s'affranchir des variations d'intensité du flux lumineux éclairant l'individu : ces variations affectent les valeurs absolues mais non les valeurs relatives et donc la distribution spatiale des niveaux de gris. Ces variations peuvent donc être a priori négligées mais pourraient être aussi bien mesurées pour apporter technologiquement les corrections nécessaires au niveau moyen de gris. Il est donc dans les deux cas possible de détecter "de manière passive" une absence de mouvement de l'individu en dépit de modifications intervenues dans les conditions d'éclairage de la scène entre deux images comparées.

Selon une caractéristique de l'invention, le procédé consiste à former sur ledit ensemble d'éléments photosensibles une image composite qui est la superposition des images de plusieurs zones d'observation déterminées d'un espace déterminé dans lequel ledit individu est susceptible de se mouvoir, et à comparer entre elles lesdites images composites successives.

Selon une autre caractéristique, lesdites zones sont réparties dans ledit espace de manière qu'en toute position de l'individu dans cet espace une partie de son corps susceptible de présenter des mouvements liés à la respiration se trouve dans au moins l'une desdites zones.

L'espace en question peut être le lit sur lequel repose un enfant. En choisissant une répartition judicieuse des zones observées et en superposant les images de ces différentes zones sur l'ensemble d'éléments photosensibles, on limite le champ d'observation et le coût de l'appareillage de surveillance tout en assurant qu'une apnée ou dyspnée pourra être détectée quelle que soit la position de l'enfant dans son lit.

De préférence, on assurera un éclairement sensiblement constant de la partie de l'individu dont une image est formée de façon à s'affranchir des variations de niveau de gris qui seraient dues à des modifications des conditions d'éclairage.

L'invention a également pour objet un dispositif de surveillance de la respiration d'un individu susceptible de se mouvoir dans un espace déterminé; ce dispositif comprend des moyens pour exposer au moins partiellement le corps dudit individu à un flux lumineux, un objectif formant une image d'au moins une zone dudit espace sur un ensemble d'éléments photosensibles, ladite zone (P₁, P₂, P₃) englobent au moins une partie du corps dudit individu susceptible de présenter des mouvements quasi périodiques liés à la respiration des circuits de pilotage et de lecture des éléments photosensibles, un convertisseur analogique-numérique, des moyens de traitement de l'information, tels qu'un microcalculateur, pour mesurer et enregistrer les niveaux de gris des points de ladite image constitués par lesdits éléments photosensibles soumis au flux lumineux retransmis par ladite zone, pour répéter ladite mesure et ledit enregistrement avec une Fréquence de récurrence ou de lecture prédéterminée pour enregistrer des images successives de ladite zone, pour comparer entre eux les niveaux de gris des points correspondants d'images successives, et pour détecter un défaut de respiration, apnée ou dyspnée, dudit individu en l'absence de variations significatives ou en présence de variations anormales de la distribution spatiale des niveaux de gris des points d'images successives comparées, et des moyens d'alarme déclenchés par lesdits moyens de traitement de l'information en réponse à la détection d'une absence de variations significatives ou d'une présence de variations anormales.

Selon une forme préférée de réalisation, le dispositif comprend des moyens optiques tels que des prismes pour former sur l'ensemble d'éléments photosensibles l'image composite qui est la superposition des images de plusieurs zones déterminées dudit espace.

Selon encore une autre caractéristique, le dispositif comporte une source lumineuse de rayonnement infrarouge du domaine non visible. En faisant "l'obscurité" dans la pièce dans laquelle l'enfant dort, cette solution permet d'assurer l'éclairement sensiblement constant du champ d'observation du dispositif sans perturber le sommeil de l'enfant.

On notera qu'il était déjà connu par un article de MEDICAL AND BIOLOGICAL ENGINEERING AND COMPUTING (vol. 24, n° 2, Mars 1986, pages 193-196, IFMBE, Stevenage, Herts, GB ; H. TEICHTAHL et al. : "Measurement of in vitro ciliary beat frequency : a television-video modification of the transmitted light technique") de mesurer la fréquence des battements de cellules ciliaires au moyen d'une caméra video, d'un microscope et d'une chaîne vidéo, en observant les mouvements des cellules ciliaires sur l'image agrandie présente à l'écran vidéo.

Néanmoins, il s'agit là d'une technique de laboratoire qui nécessite l'emploi d'un microscope à fort grossissement puisque, selon un exemple donné dans l'article, les cellules observées ont 0,2 µm de large et 6 µm de long.

Il n'était donc pas à la portée d'un spécialiste des appareils médicaux de surveillance de combiner des enseignements aussi différents que ceux de l'article de Messieurs TEICHTAHL et al. et du document FR-A-2 141 047 pour aboutir à la présente invention, et en tout état de cause cette combinaison ne suggère pas de comparer la distribution spatiale des niveaux de gris d'images successives d'une partie du corps de l'individu sous surveillance.

D'autres caractéristiques et avantages de l'invention résulteront de la description qui va suivre d'un mode de réalisation donné uniquement à titre d'exemple et illustré par les dessins annexés sur lesquels :
La figure 1 représente schématiquement l'installation du dispositif selon l'invention sur un lit d'enfant.
La figure 2 est un schéma montrant les composants essentiels du dispositif selon l'invention.
La figure 3 est une vue schématique en élévation latérale montrant la répartition des zones d'observation du dispositif de la figure 1.
Les figures 4A et 4B représentent schématiquement le déroulement dans le temps des étapes essentielles du procédé selon l'invention.

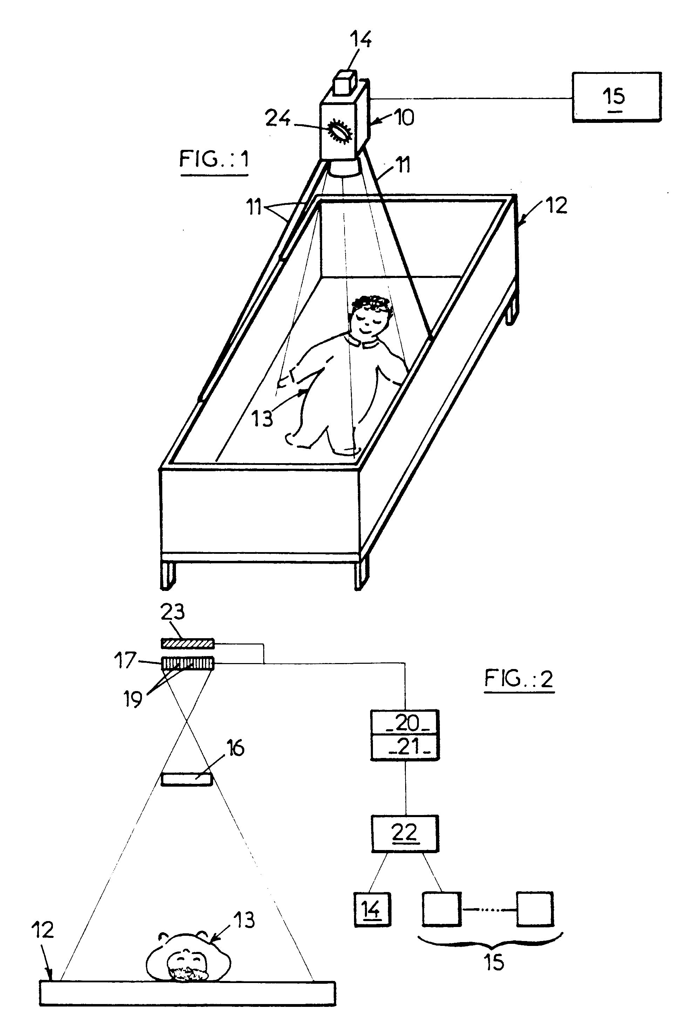

On se réfèrera d'abord à la figure 1 sur laquelle le dispositif 10 selon l'invention est fixé, par exemple, par un ensemble de pieds 11 au-dessus du lit 12 où se trouve l'enfant 13 à surveiller. Il va de soi que si la nature du lit le permet, ce dispositif 10 peut éventuellement être disposé sur le côté de celui-ci. Au dispositif 10 proprement dit sont associés un dispositif d'alarme locale lumineuse ou sonore 14 et un dispositif d'alerte à distance 15 placé dans un autre local. La liaison entre le dispositif 10 et le dispositif d'alarme à distance 15 peut être assurée par tout moyen approprié, par exemple par interphone ou par un canal de transmission d'informations spécialisé ou non (par exemple via un réseau électrique).

Les composants essentiels du dispositif 10 seront maintenant décrits en référence à la figure 2. Le dispositif 10 comprend un objectif 16, tel qu'un objectif d'appareil photographique, dans le champ d'observation duquel se trouve le lit 12 sur lequel repose l'enfant 13.

L'objectif 16 est associé à un ensemble d'éléments photosensibles constituéss de préférence par une barrette ou rangée 17 de photodétecteurs élémentaires 19 tels que des photodétecteurs à transfert de charge, qui peuvent être par exemple au nombre de 512 ou 1024.

L'objectif 16 forme sur la barrette 17 de photodétecteurs l'image d'un plan de l'espace compris dans son champ d'observation, ce plan correspondant au plan du dessin de la figure 2. Les positions de l'objectif 16 et de la barrette 17 l'un par rapport à l'autre et par rapport au lit 12 sont choisies de manière que le plan de l'espace observé coupe le plan défini par la surface du matelas du lit 12 dans une zone de celui-ci où se trouve une partie du corps de l'enfant 13 susceptible de présenter des mouvements liés à la respiration, à savoir le thorax ou l'abdomen.

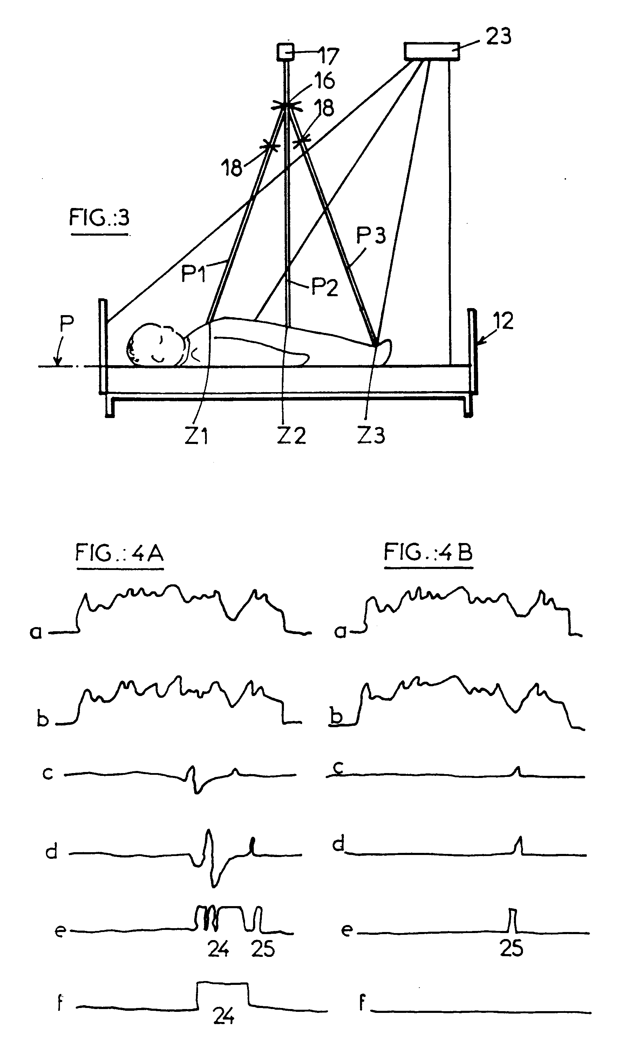

Toutefois, comme l'enfant est susceptible de se mouvoir dans son lit pendant son sommeil, il est préférable de former des images de plusieurs zones distinctes du lit 12 qui sont réparties de manière qu'en toute position de l'enfant dans son lit une image d'une partie de son thorax ou de son abdomen est formée sur la barrette 17. C'est ainsi que, selon le mode de réalisation de la figure 3, des moyens optiques complémentaires tels que des prismes 18 sont associés à l'objectif 16 pour former des images suivant trois plans P1, P2 et P3 s'étendant transversalement par rapport à la longueur du lit 12. L'optique 16,18 est réalisée de telle manière que les images de trois bandes Z1, Z2 et Z3 se superposent et donnent lieu à une image composite sur la barrette 17 de photodétecteurs.

Bien entendu, il s'agit là d'un exemple particulier de réalisation qui peut faire l'objet de nombreuses variantes, tant en ce qui concerne le nombre de plans de l'espace observé que les orientations de ces plans les uns par rapport aux autres et par rapport au plan P du matelas sur lequel repose l'enfant. Ces différents paramètres peuvent être optimisés en fonction de la taille du lit 12, de celle de l'enfant, etc... afin d'assurer qu'en toute position de celui-ci dans son lit au moins une image d'une partie de son thorax ou de son abdomen soit formée sur la barrette 17 de photodétecteurs. Toutefois, il n'y a pas lieu de multiplier à l'excès le nombre d'images superposées sur la barrette 17 car il risquerait d'en résulter une diminution excessive de la sensibilité du dispositif par superposition d'images significatives (parties du corps de l'enfant présentant dos mouvements liés à la respiration) et d'images non significatives (observation de zones du lit dans lequel ne se trouve aucune partie du corps de l'enfant ou de parties de celui-ci qui ne présentent pas de mouvements liés à la respiration). En effet, chaque photodétecteur de la barrette 17 est sensible à la moyenne des points homologues des images superposées formées dans les différents plans d'observation.

Les photodétecteurs 19 de la barrette 17 sont associés à des circuits généralement désignés par la référence 20, qui comprennent des circuits de mesure ou lecture des charges des détecteurs 19 et au moins une horloge de détermination du temps d'intégration ou de la fréquence de lecture des photodétecteurs 19. Les circuits de lecture sont reliés par un convertisseur analogique-numérique 21 à un microcalculateur 22 effectuant le traitement des signaux de sortie des photodétecteurs 19 et la commande des circuits 20.

Ce microcalculateur 22 commande également, à l'aide des circuits 20, un système d'éclairage 23 qui assure un éclairage sensiblement constant des bandes Z1, Z2 et Z3 dont des images sont formées sur la barrette 17. De préférence, le système d'éclairage 23 est constitué d'un ensemble de diodes photoémissives éclairant les zones précitées à une longueur d'onde qui ne se trouve pas dans le domaine du visible tout en restant dans le domaine de sensibilité des photodétecteurs 19. Un éclairage par infrarouge non visible a l'avantage de ne pas être nocif pour l'enfant aux intensités exigées par la sensibilité des photodétecteurs et de présenter un degré de réflexion satisfaisant sur les nombreux types de matériaux dont l'enfant 13 peut être revêtu. Si le système d'éclairage est alimenté par une source de tension alternative, la fréquence de lecture des photodétecteurs doit être égale à ou être un sous-multiple entier de deux fois la fréquence de la source de tension afin que le niveau de gris des photodétecteurs soit indépendant de la phase de la source. A titre d'exemple, une fréquence de lecture de 20 Hz est compatible avec des sources de tension à 50 Hz et 60 Hz.

Le système d'éclairage ou source lumineuse 23 peut être disposé au voisinage immédiat de la barrette de photodétecteurs 17, ou bien être quelque peu éloigné de celle-ci de manière à éclairer obliquement las différentes zones observées Z1, Z2 et Z3 afin de contribuer, par la formation d'ombres portées au niveau de ces zones, à accroître les différences de luminosité entre les images d'une même zone qui sont formées au cours d'un cycle de respiration de l'enfant. On notera à ce sujet que de préférence l'enfant ne sera pas recouvert d'une couverture ou vêtement qui soit susceptible de rendre non perceptibles les mouvements de son corps liés à la respiration. Il pourra être vêtu, par exemple, d'un vêtement moulant. D'autre part, en l'absence de système d'éclairage spécifique, la source lumineuse peut être constituée par la lumière naturelle. Dans ce cas, des moyens d'asservissement de sensibilité peuvent être prévus pour que le niveau de gris moyen vu par les photodétecteurs soit sensiblement constant et indépendant des conditions d'éclairage.

Par ailleurs, le microprocesseur 22 est relié au dispositif d'alarme lumineux ou sonore 14 associé au dispositif ainsi qu'au système d'alerte à distance 15. Ces moyens d'alarme locale ou d'alerte à distance sont déclenchés par le microcalculateur 22 lorsqu'il ne détecte plus de respiration.

On va maintenant décrire les principales étapes du procédé selon l'invention, ainsi que le fonctionnement du dispositif en référence aux figures 4A et 4B.

Les courbes a et b des figures 4A et 4B représentent les signaux vidéo obtenus par lecture des photodétecteurs élémentaires 19 de la barrette 17 à deux instants qui sont séparés d'un intervalle de temps prédéterminé.

Chaque courbe a, b représente les niveaux de gris qui sont vus par l'ensemble des photodétecteurs 19. Lorsque la scène observée n'a pas changé entre les deux instants d'observation, les courbes a et b sont identiques (figure 4B), l'éclairage étant supposé sensiblement constant. Par contre, lorsqu'il y a un mouvement respiratoire, ceci se traduit par une variation de la distribution spatiale des niveaux de gris vus par les photodétecteurs correspondants (figure 4A). La détection des variations de la distribution spatiale des niveaux de gris vus par les photodétecteurs permet donc de mettre en évidence les mouvements du corps liés à la respiration et, en corollaire, l'absence de ces mouvements ou leur caractère anormal permet de déclencher l'alerte.

Le traitement des signaux vidéo fournis par la barrette 17 de photodétecteurs 19 est, pour l'essentiel, le suivant :
deux signaux successifs tels que ceux représentés par les courbes a et b sont comparés, et leur différence est effectuée pour obtenir le signal représenté par la courbe c. Ce signal est amplifié (courbe d) puis est redressé et transformé en signaux binaires par comparaison à un seuil déterminé parmettant d'éliminer le bruit résiduel des photodétecteurs 19. Le signal résultant, représenté par la courbe e, comprend un premier créneau ou écho 24, de largeur relativement importante (figure 4A), et un second créneau ou écho 25 de largeur beaucoup plus faible. Ce signal est ensuite appliqué à un filtre, pour ne conserver que le premier écho 24 (courbe f) qui correspond à un mouvement lié à la respiration, et pour éliminer le second écho 25, dont la longueur est beaucoup trop faible pour correspondre à un tel mouvement (cas d'un insecte se déplaçant dans le champ d'observation).

De façon plus détaillée, le déroulement dans le temps des diverses opérations du procédé selon l'invention peut être le suivant :
Les images formées sur la barrette 17 de photodétecteurs sont lues à une fréquence déterminée et les charges électriques de l'ensemble des photodétecteurs 19 explorés successivement produisent las signaux vidéo a et b des figures 4A et 4B. Ces signaux lus par le circuit 20 et numérisés par le convertisseur 21 sont enregistrés par le microcalculateur 22 à une fréquence qui est au plus égale à la fréquence de lecture et qui est le plus souvent Un sous-multiple de cette fréquence de lecture.

Supposons par exemple que cet intervalle de temps soit de 3 secondes et que la fréquence d'enregistrement soit telle que l'on ait un enregistrement toutes les 0,5 seconde. En d'autres termes, le premier signal enregistré à l'instant 0 est comparé 3 secondes plus tard au signal qui vient d'être enregistré et leur différence est traitée comme décrit en référence à la figure 4 (amplification, redressement, transformation en signaux binaires et filtrage pour donner le signal de résultat qui est enregistré à l'instant t = 3 secondes. Le signal enregistré au temps t = 0,5 seconde est comparé au signal enregistré au temps t = 3,5 secondes, et ainsi de suite:

L'étape suivante consiste, lorsqu'un résultat est négatif (pas de respiration) à accumuler les résultats suivants un nombre prédéterminé de fois ou un temps prédéterminé et de donner l'alarme après un nombre consécutif et prédéterminé de résultats négatifs signifiant que l'apnée ne correspond pas à une pause mais à un arrêt respiratoire. Ce nombre cumulé d'images statiques correspond à la durée de la pause respiratoire acceptable qui peut, soit être déterminée empiriquement et mémorisée dans le microcalculateur 22, soit réglée au moyen d'un sélecteur à commande manuelle 24 prévu sur le dispositif 10 (figure 1).

Il va de soi que les modes de réalisation décrits ne sont que des exemples et l'on pourrait les modifier, notamment par substitution d'équivalents techniques, sans sortir pour cela du cadre de l'invention.

## Revendications

1. Procédé de surveillance de la respiration d'un individu, suivant lequel le corps dudit individu est au moins partiellement soumis à un champ, les perturbations causées au champ par les mouvements de l'individu sont détectées et une alarme est déclenchée lorsque le degré de mouvement perturbant le champ tombe au-dessous d'un seuil prédéterminé pendant un temps supérieur à une valeur prédéterminée, ledit procédé étant caractérisé en ce qu'il consiste à exposer au moins partiellement le corps dudit individu à un flux lumineux, à former sur un ensemble d'éléments photosensibles (17) l'image d'au moins une zone (P1, P2, P3) de l'espace exposée au flux lumineux et comprise dans le champ d'observation d'un moyen optique (10) associé aux éléments photosensibles (17), ladite zone (P1, P2, P3) englobant au moins une partie du corps dudit individu (13) susceptible de présenter des mouvements quasi périodiques liés à la respiration, à mesurer et enregistrer les niveaux de gris des points de ladite image constitués par lesdits éléments photosensibles soumis au flux lumineux retransmis par ladite zone, à répéter ladite mesure et ledit enregistrement avec une fréquence de récurrence ou de lecture prédéterminée pour enregistrer des images successives de ladite zone, à comparer entre eux les niveaux de gris des points correspondants d'images successives, et à détecter un défaut de respiration, apnée ou dyspnée, dudit individu en l'absence de variations significatives ou en présence de variations anormales de la distribution spatiale des niveaux de gris des points d'images successives comparées.

2. Procédé selon la revendication 1, caractérisé en ce qu'il consiste à former sur ledit ensemble d'éléments photosensibles (17) une image composite qui est la superposition des images de plusieurs zones d'observation déterminées (P1, P2, P3) d'un espace déterminé (12) dans lequel ledit individu est susceptible de se mouvoir, et à comparer entre elles des images composites successives.

3. Procédé selon la revendication 2, caractérisé en ce que lesdites zones (P1, P2, P3) sont réparties dans ledit espace (12) de manière qu'en toute position de l'individu (13) dans cet espace une partie de son corps susceptible de présenter des mouvements liés à la respiration se trouve dans au moins l'une desdites zones.

4. Procédé selon l'une quelconque des revendications 1 à 3, caractérisé en ce qu'il consiste à enregistrer des images successives à une fréquence qui est un sous-multiple de la fréquence de lecture et à comparer entre elles deux à deux des images successives enregistrées séparées l'une de l'autre par un nombre déterminé d'images enregistrées.

5. Procédé selon la revendication 4, caractérisé en ce qu'il consiste à former un signal électrique analogique représentatif du niveau de gris de tous les points de chaque image enregistrée, à comparer les signaux électriques de deux images successives enregistrées, et à détecter un défaut de respiration en l'absence de différence significative entre les amplitudes des signaux comparés.

6. Procédé selon la revendication 5, caractérisé en ce qu'il consiste à effectuer la différence entre les signaux électriques comparés, à amplifier, redresser et convertir sous forme numérique, par comparaison à un seuil déterminé, le signal différence obtenu, et à filtrer ledit signal numérique pour éliminer les signaux de dimension trop faible pour correspondre à un mouvement respiratoire.

7. Procédé selon l'une quelconque des revendications 4 à 6, caractérisé en ce qu'il consiste à comparer entre eux un nombre prédéterminé de résultats de comparaisons successives pour éliminer des résultats erronés dus, en particulier, à des pauses du cycle respiratoire dudit individu.

8. Procédé selon l'une quelconque des revendications 4 à 7, caractérisé en ce qu'il consiste à comparer plusieurs images successives au cours de chaque cycle respiratoire de l'individu.

9. Procédé selon l'une quelconque des revendications 1 à 8, caractérisé en ce qu'il consiste à assurer un éclairement sensiblement constant de ladite partie de l'individu dont une image est formée.

10. Procédé selon la revendication 9, caractérise en ce que dans le cas où on assure ledit éclairement au moyen d'une source lumineuse alimentée par une source de tension alternative, ladite fréquence de lecture est égale à ou est un sous-multiple entier de deux fois la fréquence de la source de tension alternative.

11. Dispositif de surveillance de la respiration d'un individu susceptible de se mouvoir dans un espace déterminé, caractérisé en ce qu'il comprend des moyens pour exposer au moins partiellement le corps dudit individu à un flux lumineux, un objectif (16) formant une image d'au moins une zone (P1, P2, P3) dudit espace sur un ensemble (17) d'éléments photosensibles (19), ladite zone (P₁, P₂, P₃) englobant au moins une partie du corps dudit individu susceptible de présenter des mouvements quasi périodiques liés à la respiration des circuits (20) de pilotage et de lecture des éléments photosensibles, un convertisseur analogique-numérique (21), des moyens (22) de traitement de l'information, tels qu'un microcalculateur, pour mesurer et enregistrer les niveaux de gris des points de ladite image constitués par lesdits éléments photosensibles soumis au flux lumineux retransmis par ladite zone, pour répéter ladite mesure et ledit enregistrement avec une Fréquence de récurrence ou de lecture prédéterminée pour enregistrer des images successives de ladite zone, pour comparer entre eux les niveaux de gris des points correspondants d'images successives, et pour détecter un défaut de respiration, apnée ou dyspnée, dudit individu en l'absence de variations significatives ou en présence de variations anormales de la distribution spatiale des niveaux de gris des points d'images successives comparées, et des moyens d'alarme (14, 15) déclenchés par lesdits moyens de traitement de l'information en réponse à la détection d'une absence de variations significatives ou d'une présence de variations anormales.

12. Dispositif selon la revendication 11, caractérisé en ce qu'il comprend des moyens pour enregistrer les signaux fournis par les éléments photosensibles à une fréquence qui est un sous-multiple de la fréquence de lecture des éléments photosensibles, et des moyens pour comparer deux à deux des signaux enregistrés successifs séparés l'un de l'autre par un nombre déterminé de signaux et pour effectuer leur différence.

13. Dispositif selon la revendication 12, caractérisé en ce qu'il comprend des circuits de transformation en signaux binaires des différences des signaux comparés et des circuits de filtrage pour l'élimination des différences non significatives.

14. Dispositif selon l'une des revendications 11 à 13, caractérisé en ce qu'il comprend des moyens de comparaison d'un nombre déterminé de résultats obtenus successifs et d'élimination des résultats aberrants.

15. Dispositif selon l'une quelconque des revendications 11 à 14, caractérisé en ce qu'il comprend des moyens optiques (18) pour former sur ledit ensemble (17) d'éléments photosensibles une image composite qui est la superposition des images de plusieurs zones déterminées (P1, P2, P3) dudit espace.

16. Dispositif selon la revendication 15, caractérisé en ce que lesdits moyens optiques comprennent des prismes (18).

17. Dispositif selon l'une quelconque des revendications 11 à 17, caractérisé en ce qu'il comporte une source lumineuse (23) de rayonnement infrarouge du domaine non visible.

## Claims

1. Method for monitoring the respiration of an individual, in accordance with which the body of the said individual is subjected at least partially to a field, the disturbances caused to the field by the movements of the individual are detected and an alarm is triggered when the degree of movement disturbing the field falls below a predetermined threshold for a peroid of time greater than a predetermined value, the said method being characterized in that it consists in exposing the body of the said individual at least partially to a luminous flux, in forming on a set of photosensitive elements (17) the image of at least one zone (P₁, P₂, P₃) of the space exposed to said luminous flux and comprised in the observation field of an aptical mean (10) associated to said photosensitive elements (17), said zone (P₁, P₂, P₃) including at least one part of the body of the said individual (13) liable to exhibit quasi-periodic movements associated with breathing, in measuring and recording the gray levels of the points of said image constituted by said photosensitive elements subjected to the luminous flux transmitted back by said zone, in repeating said measure and said record at a predetermined recurrence or reading frequency for recording successive images of said zone, in comparing the gray levels of the corresponding points of successives images, and in detecting a respiratory insufficiency, apnoea or dyspnoea, of the said individual in the absence of significant variations or in the presence of abnormal variations in the spatial distribution of the gray levels of the points of successive images compared.

2. Method comparing according to claim 1, characterized in that it consists in forming on the said set of photosensitive elements (17) a composite image which is the superposition of the images of several determined observation zones (P₁, P₂, P₃) of a determined space (12) in which the said individual is liable to move, and in comparing successive composite images.

3. Method according to claim 1, characterized in that the said zone (P₁, P₂, P₃) are distributed in the said space (12) in such a way that, in any position of the individual (13) within this space, a part of his or her body liable to show movements associated with breathing is situated in at least one of the said zones.

4. Method according to any one of claims 1 to 3, characterized in that it consists in recording successive images at a frequency which is a sub-multiple of the reading frequency and in comparing in pairs successive recorded images separated one from the other by a determined number of recorded images

5. Method according to Claim 4, characterized in that it consists in forming an analog electrical signal representing the gray level of all the points of each recorded image, in comparing the electrical signals of two recorded successive images, and in detecting a respiratory insufficiency in the absence of a significant difference between the amplitudes of the signals compared.

6. Method according to Claim 5, characterized in that it consists in establishing the difference between the electrical signals compared, in amplifying, straightening and converting in numerical form, by comparison with a determined threshold, the signal difference obtained, and in filtering the said numerical signal in order to eliminate the signal which are of too small a dimension to correspond to a respiratory movement.

7. Method according to any one of Claims 4 to 6, characterized in that it consists in comparing a predetermined number of results of successive comparisons in order to eliminate erroneous results due, in particular, to pauses in the respiratory cycle of the said individual.

8. Method according to any one of Claims 4 to 7, characterized in that it consists in comparing several successive images during each respiratory cycle of the individual.

9. Method according to any one of Claims 1 to 8, characterized in that it consists in providing substantially constant illumination of the said part of the individual of which an image is formed.

10. Method according to Claim 9, characterized in that in the case in which the said illumination is provided by means of a luminous source supplied by a alternating voltage source, the said reading frequency is equal to or is an integral sub-multiple of twice the frequency of the alternating voltage source.

11. Device for monitoring the respiration of an individual liable to move within a determined space, characterized in that it comprises means for exposing the body of said individual at least partially to a luminous flux, a lens (16) forming an image of at least one zone (P1, P2, P3) of said space on a set (17) of photosensitive elements (19), said zone (P1, P2, P3) including at least one part of the body of said individual liable to exhibit quasi-periodic movements associated with breathing, circuits (20) for controlling and reading the photosensitive elements, an analog to digital converter (21), information-processing means (22), such as a microprocessor, for measuring and recording the gray levels of the points of said image constituted by said photosensitive elements subjected to the luminous flux transmitted back by said zone, for repeating said measure and said record at a predetermined recurrence or reading frequency for recording successive images of said zone, for comparing the gray levels of the corresponding points of successive images, and for detecting a respiratory insufficiency, apnoea or dyspnoea, of said individual in the absence of significant variations or in the presence of abnormal variations in the spatial distribution of the gray levels of the points of successive images compared, and alarm means (14,15) triggered by the said information-processing means in response to the detection of an absence of significant variations or the presence of abnormal variations.

12. Device according to Claim 11, characterized in that it comprises means for recording the signals supplied by the photosensitive elements at a frequency which is a sub-multiple of the reading frequency of the photosensitive elements, and means for comparing in pairs successive recorded signals separated one from the other by a determined number of signals and for establishing their difference.

13. Device according to Claim 12, characterized in that it comprises circuits for conversion into binary signals of the differences of the signals compared, and filter networks for eliminating non-significant differences.

14. Device according to any one of Claims 11 to 13, characterized in that it comprises means for comparing a determined number of successive results obtained and for eliminating erroneous results.

15. Device according to any one of Claims 11 to 14, characterized in that it comprises optical means (18) for forming on the said set (17) of photosensitive elements a composite image which is the superposition of the images of several determined zones (P1, P2, P3) of the said space.

16. Device according to Claim 15, characterized in that the said optical means comprise prisms (18).

17. Device according to any one of Claims 11 to 17, characterized in that it comprises a luminous source (23) of infrared radiation in the non-visible range.

## Patentansprüche

1. Verfahren zum Überwachen der Atmung eines Individuums, das zumindest teilweise einem Feld ausgesetzt ist, bei dem die durch die Bewegungen des Individuums verursachten Störungen des Feldes detektiert werden und ein Alarmsignal ausgelöst wird, wenn das Ausmaß der das Feld störenden Bewegung während einer Zeitdauer, die über einem vorgegebenen Wert liegt, unter eine vorgegebene Schwelle fällt, dadurch gekennzeichnet, daß man den Körper des Individuums mindestens teilweise einem Lichtstrom aussetzt, daß man auf einer Anordnung von lichtempfindlichen Elementen (17) ein Bild von mindestens einer Zone (P1, P2, P3) des dem Lichtstrom ausgesetzten Raumes, die im Beobachtungsfeld einer den lichtempfindlichen Elementen (17) zugeordneten optischen Einrichtung (10) liegt, erzeugt, wobei diese Zone (P1, P2, P3) mindestens einen Teil des Körpers des Individuums (13) umfaßt, der zu mit der Atmung verknüpften quasiperiodischen Bewegungen fähig ist, die Graupegel von Punkten des durch die lichtempfindlichen Elemente, die dem von der erwähnten Zone zurückübertragenen Sichtstrom ausgesetzt waren, erzeugten Bildes mißt und aufzeichnet, diese Messung und Aufzeichnung mit einer vorgegebenen Folge- oder Abtastfrequenz wiederholt, um aufeinanderfolgende Bilder dieser Zone aufzuzeichnen, die Graupegel der den aufeinanderfolgenden Bildern entsprechenden Punkte miteinander vergleicht und einen Atmungsfehler des Individuums, entweder Atmungsstillstand oder Atmungsnot, bei Fehlen von signifikanten Schwankungen oder bei Vorhandensein von unnormalen Schwankungen der räumlichen Verteilung der Graupegel der Punkte der miteinander verglichen aufeinanderfolgenden Bilder detektiert.

2. Verfahren nach Anspruch 1, dadurch gekennzeichnet, daß man auf der Anordnung der lichtempfindlichen Elemente (17) ein zusammengesetztes Bild erzeugt, das aus einer Überlagerung der Bilder von einer Vielzahl von bestimmten Beobachtungszonen (P1, P2, P3) in einem festgelegten Raum (12), in dem sich das Individuum bewegen kann, besteht, und daß man aufeinanderfolgende zusammengesetzte Bilder untereinander vergleicht.

3. Verfahren nach Anspruch 2, dadurch gekennzeichnet, daß die Zonen (P1, P2, P3) derart in dem Raum (12) verteilt sind, daß sich in jeder Position des Individuums (13) in diesem Raum ein Teil seines Körpers, der zu den mit der Atmung verknüpften Bewegungen fähig ist, in mindestens einer der erwähnten Zonen befindet.

4. Verfahren nach einem der Ansprüche 1 bis 3, dadurch gekennzeichnet, daß man die aufeinanderfolgenden Bilder mit einer Frequenz aufzeichnet, die einem Teilvielfachen der Abtastfrequenz entspricht, und daß man die aufeinanderfolgenden aufgezeichneten Bilder paarweise untereinander eines vom anderen getrennt über eine festgelegte Zahl an aufgezeichneten Bildern vergleicht.

5. Verfahren nach Anspruch 4, dadurch gekennzeichnet, daß man ein elektrisches Analogsignal erzeugt, das den Graupegel aller Punkte eines jeden aufgezeichneten Bildes repräsentiert, daß man die elektrischen Signale von zwei aufeinanderfolgenden aufgezeichneten Bildern vergleicht und daß man einen Atmungsfehler bei Fehlen einer signifikanten Differenz zwischen den Amplituden der verglichenen Signale detektiert.

6. Verfahren nach Anspruch 5, dadurch gekennzeichnet, daß man die Differenz zwischen den verglichenen elektrischen Signalen ermittelt, die erhaltene Signaldifferenz verstärkt, gleichrichtet und durch Vergleich mit einem vorgegebenen Schwellenwert in die numerische Form umwandelt und das numererische Signal filtert, um Signale einer zu geringen Größe, um einer Atmungsbewegung zu entsprechen, zu eliminieren.

7. Verfahren nach einem der Ansprüche 4 bis 6, dadurch gekennzeichnet, daß man eine vorgegebene Anzahl von aufeinanderfolgenden Vergleichsergebnissen miteinander vergleicht, um fehlerhafte Ergebnisse zu eliminieren, insbesondere in den Pausen des Atmungszyklus des Individuums.

8. Verfahren nach einem der Ansprüche 4 bis 7, dadurch gekennzeichnet, daß man mehrere aufeinanderfolgende Bilder im Laufe eines jeden Atmungszyklus des Individuums vergleicht.

9. Verfahren nach einem der Ansprüche 1 bis 8, dadurch gekennzeichnet, daß man eine Beleuchtung mit konstanter Empfindlichkeit des Teiles des Individuums, von dem ein Bild erzeugt wird, sicherstellt.

10. Verfahren nach Anspruch 9, dadurch gekennzeichnet, daß in dem Fall, in dem die Beleuchtung mit Hilfe einer durch eine Wechselstromquelle gespeisten Lichtquelle sichergestellt wird, die Abtastfrequenz der doppelten Frequenz der Wechselstromquelle oder einem Teilvielfachen davon entspricht.

11. Vorrichtung zur Überwachung der Atmung eines Individuums, das sich in einem festgelegten Raum bewegen kann, dadurch gekennzeichnet, daß sie Einrichtungen, um den Körper des Individuums zumindest teilweise einem Lichtstrom auszusetzen, ein Objektiv (16), das ein Bild von mindestens einer Zone (P1, P2, P3) des Raumes auf einer Anordnung (17) von lichtempfindlichen Elementen (19) erzeugt, wobei diese Zone (P1, P2, P3) mindestens einen Teil des Körpers des Individuums umfaßt, der mit der Atmung verknüpfte quasiperiodische Bewegungen erzeugen kann, Schaltungen (20) zum Steuern und Abtasten der lichtempfindlichen Elemente, einen A/D-Umformer (21), Einrichtungen (22) zur Verarbeitung von Informationen, wie beispielsweise einen Mikroprozessor, um die Graupegel von Punkten des Bildes zu messen und aufzuzeichnen, das von den lichtempfindlichen Elementen erzeugt wurde, welche dem von der Zone zurückübertragenen Lichtstrom ausgesetzt waren, um diese Messung und diese Aufzeichnung mit einer vorgegebenen Folgefrequenz oder Abtastfrequenz zu wiederholen, um aufeinanderfolgende Bilder dieser Zone aufzuzeichnen, um die Graupegel von entsprechenden Punkten von aufeinanderfolgenden Bildern miteinander zu vergleichen und um einen Atmungsfehler, entweder Atmungsstillstand oder Atmungsnot, des Individuums bei Fehlen von signifikanten Schwankungen oder beim Vorhandensein von unnormalen Schwankungen in bezug auf die räumliche Verteilung der Graupegel der Punkte von verglichenen aufeinanderfolgenden Bildern zu detektieren, und Alarmeinrichtungen (14, 15) umfaßt, die durch die Informationsverarbeitungseinrichtungen in Abhängigkeit vom Erfassen des Fehlens von signifikanten Schwankungen oder des Vorhandenseins von unnormalen Schwankungen ausgelöst werden.

12. Vorrichtung nach Anspruch 11, dadurch gekennzeichnet, daß sie Einrichtungen zum Aufzeichnen der von den lichtempfindlichen Elementen gelieferten Signale mit einer Frequenz, die ein Teilvielfaches der Abtastfrequenz der lichtempfindlichen Elemente ist, und Einrichtungen zum paarweisen Vergleichen von aufeinanderfolgenden aufgezeichneten Signalen in voneinander getrennter Weise über eine bestimmte Signalzahl und zum Ermitteln ihrer Differenz umfaßt.

13. Vorrichtung nach Anspruch 12, dadurch gekennzeichnet, daß sie Schaltungen zur Umformung der Differenzen der verglichenen Signale in Binärsignale und Filterschaltungen zum Eliminieren von Differenzen, die nicht signifikant sind, umfaßt.

14. Vorrichtung nach einem der Ansprüche 11 bis 13, dadurch gekennzeichnet, daß sie Einrichtungen zum Vergleichen einer bestimmten Zahl von aufeinanderfolgenden erhaltenen Ergebnissen und zum Eliminieren von abweichenden Ergebnissen umfaßt.

15. Vorrichtung nach einem der Ansprüche 11 bis 14, dadurch gekennzeichnet, daß sie optische Einrichtungen (18) zur Ausbildung eines zusammengesetzten Bildes auf der Anordnung (17) der lichtempfindlichen Elemente aufweist, wobei das zusammengesetzte Bild einer Überlagerung von Bildern von mehreren bestimmten Zonen (P1, P2, P3) des Raumes entspricht.

16. Vorrichtung nach Anspruch 15, dadurch gekennzeichnet, daß die optischen Einrichtungen Prismen (18) umfassen.

17. Vorrichtung nach einem der Ansprüche 11 bis 16, dadurch gekennzeichnet, daß sie eine Infrarotlichtquelle (23) im nicht sichtbaren Bereich aufweist.
